Europäisches Patentamt

European Patent Office

Office européen des brevets

(11) Publication number: **0 290 781**
**A2**

# EUROPEAN PATENT APPLICATION

(21) Application number: **88105468.8**

(22) Date of filing: **06.04.88**

(51) Int. Cl.4: **C12M 1/04**

(30) Priority: **01.05.87 US 44690**

(43) Date of publication of application:
**17.11.88 Bulletin 88/46**

(84) Designated Contracting States:
**AT BE CH DE ES FR GB GR IT LI NL SE**

(71) Applicant: **Becton Dickinson and Company**
**One Becton Drive**
**Franklin Lakes New Jersey 07417-1880(US)**

(72) Inventor: **Stitt, David T.**
**32000 York Road**
**Parkton §Maryland(US)**
Inventor: **Monthony, James F.**
**13 Thaxton Court**
**Timonium Maryland(US)**
Inventor: **Hardy, Denise D.**
**6602 Eberle Dr. Apt. 202**
**Baltimore Maryland(US)**

(74) Representative: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Method for modifying a contained gaseous mixture.**

(57) Compositions are provided for modifying the atmosphere within a closed container to produce an environment suitable for the growth or maintenance of capnophilic, microaerophilic and anaerobic microorganisms. The compositions may be enclosed within a gas permeable sachet disposed inside a gas impermeable, sealable container. Some of the compositions utilize an insoluble carbonate to produce carbon dioxide, an oxygen reactive powdered metal to reduce oxygen and a powdered material coated with catalyst to accelerate the rate of oxygen reduction.

Fig. 1

# METHOD FOR MODIFYING A CONTAINED GASEOUS MIXTURE

## FIELD OF INVENTION

The present invention relates generally to a composition and method for the rapid generation of an anaerobic, microaerophilic or capneic atmosphere which is conducive to the growth of certain microorganisms. More particularly, the present invention relates to compositions and a method for modifying and controlling various components of a gaseous mixture to provide an atmosphere conducive to growth of microorganisms.

## DESCRIPTION OF THE PRIOR ART

It is well known that some microorganisms require an aerobic atmosphere for growth, others require an anaerobic atmosphere, and still others require a microaerophilic atmosphere in which the oxygen level is between aerobic and anaerobic. In addition, some microorganisms require elevated levels of carbon dioxide for enhanced growth. These microorganisms are said to be capnophilic.

The attainment of an aerobic atmosphere is relatively simple, in most cases merely requiring aeration of the culture media. Anaerobic conditions are more difficult to attain and the prior art contains many devices and processes for producing anaerobic atmospheres. U.S. Pat. No. 4,588,561 to Aswell et al describes a gas permeable sachet in a sealable envelope with a compartment in fluid communication with the sachet. The sachet contains an oxygen reactive material, a carbon dioxide generating composition, a filler and a water dispersive agent (surfactant). The sachet is inactive until the addition of water, at which time the oxygen is removed and carbon dioxide is generated. The oxygen reactive reagent consists of iron powder, copper powder or aluminum powder. The carbon dioxide generating composition is a water soluble solid acid and a water soluble carbonate. The filler consists of diatomaceous earth, charcoal, cellulose fiber, silica gel or barium sulfate. Great Britain Pat. No. 2,109,406 to Kasugai describes a process for culturing anaerobic bacteria wherein an agent which is reactive with oxygen is enclosed in an air-tight closed vessel. The agent contains a composition which is capable of removing oxygen and generating carbon dioxide in a volumetric ratio of about approximately twice as much oxygen removed as carbon dioxide generated. The preferred oxygen reactive material disclosed in the Kasugai patent is powdered iron. One embodiment of the present invention is an improvement over the oxygen removal systems described in the Aswell et al patent and the Kasugai patent and relates to compositions and methods for the rapid generation of anaerobic or microaerophilic atmospheres in a variety of disposable or reusable containers and may be used for either the growth or the maintenance of microorganisms. The inventions have significant improvements in both performance and format.

U.S. Pat. No. 3,246,959, to Brewer discloses a device for generating such anaerobic atmospheres by generation of hydrogen for reaction with oxygen in the atmosphere of a gas-tight apparatus such as that disclosed in U.S. Pat. No. 3,484,089 to Brewer. The reaction between the hydrogen and oxygen is catalyzed by a palladium catalyst in the anaerobic apparatus. It is recommended that at least one gram of alumina pellets coated with 0.5% palladium is used per liter volume of container (Oxoid Gas Generating Kit Package Insert, Oxoid Ltd., Basingstoke, Hampshire, England). Means are provided to dissipate heat generated by the reaction.

U.S. Pat. No. 4,347,222 to Beall discloses disposition of the catalyst in one receptacle of a unitary apparatus. This apparatus requires means for puncturing seals between several of the receptacles. The puncture means is supplied by a device which is separate from the gas generating apparatus or as part of a mated container.

U.S. Pat. No. 4,013,422 to Spinner discloses a container having a material for generating a reducing gas, such as hydrogen, for reaction, in the presence of a catalyst, with oxygen. The Spinner apparatus, however, uses an exposed catalyst pellet with no provision for heat removal. In addition, it relies on the breaking of an ampoule containing a liquid and thereby instantaneous release of the liquid into contact with the material for generating the reducing gas. No means are provided for controlling the rate at which the contact is made. Experience has shown that contact at a slow and controlled rate is essential for accurate attainment of predetermined final oxygen levels, particularly in those cases where it is desired to reduce, but not eliminate, the oxygen in the atmosphere.

U.S. Pat. No. 4,287,306 to Brewer describes a further apparatus for generating anaerobic atmospheres. In accordance with this patent, a flexible sealed package is provided with a catalyst coated onto the exterior surface of the package for use in catalyzing the reaction between oxygen outside the package and hydrogen generated within the package. This apparatus, like the Spinner apparatus, has the disadvantage of exposed active catalyst.

U.S. Pat. No. 4,562,051 to Darner and Stoermer teaches use of a separate safety shielded catalyst container with provisions to dissipate heat. U.S. Pat. No. 4,289,855 to Whitley also discloses a safety catalyst package designed to reduce the danger of flashing or explosion. The Whitley package encloses a catalyst within holes and folds in a metal foil net. The net, which is inside of a container having holes for gas exchange, is composed of a heat conducting material, and thereby functions to remove heat from the catalyst vicinity. The Whitley disclosure is of a safety catalyst package only, and makes no provision for supply of hydrogen.

A package, for use in an anaerobic jar and specifically designed for attainment of a microaerophilic atmosphere, is disclosed in U.S. Pat. No. 4,377,554 to Johnson. The Johnson invention generates less hydrogen and relies on control of "wetover" and "condensation" times for successful generation of microaerophilic atmospheres, and uses conventional catalysts attached either to the outside of the package or to the lid of the jar.

A package for production of a carbon dioxide enriched atmosphere in an anaerobic jar is commercially available from the BBL Microbiology Systems Division of Becton Dickinson Company. The package consists of a sodium bicarbonate plus citric acid tablet in a moisture impermeable envelope. The envelope is employed for the cultivation of aerobic microorganisms and tissue cultures which benefit from a capneic atmosphere. The envelope is also suitable for aerotolerance testing to distinguish between faculative and strict anaerobes.

A cardboard package for use in generating an anaerobic atmosphere is commercially available from the BBL Microbiology Systems Division of Becton Dickinson Company. This package includes a catalyst chamber mounted in the side of the box. The catalyst chamber is porous on both sides to permit generated hydrogen to flow through the chamber and thus react with the catalyst.

## SUMMARY OF THE INVENTION

In accordance with some features of the present invention, there is provided a gas and moisture permeable sachet or pellet containing a composition suitable for use in modifying the atmosphere within a gas impermeable package or container to support the environmental needs of a wide spectrum of microorganisms.

In accordance with one embodiment of the invention, a water insoluble carbonate is used when a carbon dioxide generating composition is desired. It is not obvious that a water insoluble carbonate would work in an aqueous environment. The use of a water insoluble carbonate causes a major change in the stability and shelf-life of the carbon dioxide generating composition.

In accordance with a further embodiment of the present invention, an admixture of an oxygen reactive material, preferably a powdered metal, and a water insoluble carbonate are provided when an atmosphere appropriate for microaerophilic organisms is required. Within one hour the composition generates growth stimulating carbon dioxide and rapidly removes toxic oxygen, leaving enough to support growth of these organisms.

In accordance with a further embodiment of the invention, a powdered catalyst is included with the oxygen reactive material. The catalyst accelerates the removal of oxygen in the atmosphere by reacting it with a by-product of the oxygen reactive material and acidified water. This effect is unanticipated as current art teaches that a catalyst is relatively inactive when wet and because no special provisions are required to dissipate heat or prevent ignitions. An anaerobic atmosphere also provides an excellent environment for the transport of aerobic, facultative anaerobic and anaerobic organisms because the organisms are protected from oxidation.

In use, one or more petri dishes having a prepared media and having been inoculated with a sample suspected of containing microorganisms are inserted into a gas impermeable container adjacent to the sachet or pellet containing a composition of the invention. A pipette or a vial is then used to dispense a predetermined level of water into the container depending on the composition. The water wets the composition and causes generation of carbon dioxide or depletion of oxygen or both. The container is then sealed by suitable means.

In a preferred embodiment of the invention the container is a flexible envelope and the oxygen reactive material is powdered iron. The powdered catalyst is preferably palladium catalyst on an inert support. The oxygen reacts with the powdered iron to form iron oxide. The acidic water reacts with the iron powder to generate hydrogen gas. The hydrogen by-product reacts with the powdered catalyst and oxygen to form water After a suitable period of time has elapsed the oxygen level contained within the envelope is reduced to a level appropriate for the growth of anaerobic organisms, e.g. less than about 2 percent of oxygen by volume or microaerophilic organisms, e.g. less than 12 percent of oxygen by volume. The catalyst reduces the time required to reach the level of oxygen appropriate for anaerobic organisms.

The present invention provides an extremely simple, rapid, easily used, self contained device for generation of a carbon dioxide atmosphere or a reduced oxygen atmosphere without the hazards associated with the conventional use of hydrogen and a catalyst.

## BRIEF DESCRIPTION OF THE DRAWINGS

Fig. 1 is an exploded view of a package which is exemplary of a container which can be used with the compositions of the invention showing the sequence of assembling the package.

Fig. 2 is a front view of the assembled package.

Fig. 3 is a side view of the assembled package.

Fig. 4 is a view of a water containing vial showing the arrangement of the tear strip used to seal the end of the vial.

Figure 5 is a graph of the carbon dioxide gas levels produced 2 hours after activation with water of sachets such as those described in the Aswell et al patent utilizing sodium carbonate. The sachets were taken at intervals during the manufacturing and packaging operations for a commercial embodiment of the sachet of the Aswell et al patent. The gas levels were determined using the standard method described in Example I.

Figure 6 is a graph of the carbon dioxide gas levels produced 2 hours after activation with water of sachets made with an insoluble carbonate formulation developed in accordance with the invention. These sachets were stored under ambient conditions without any protection. The gas levels were determined using the standard method described in Example I.

Figure 7 shows oxygen removal after activation with water for sachets having powdered iron as an oxygen removal material and which contained or did not contain a powdered catalyst. The gas levels were determined using the standard method described in Example I.

## DETAILED DESCRIPTION OF THE INVENTION

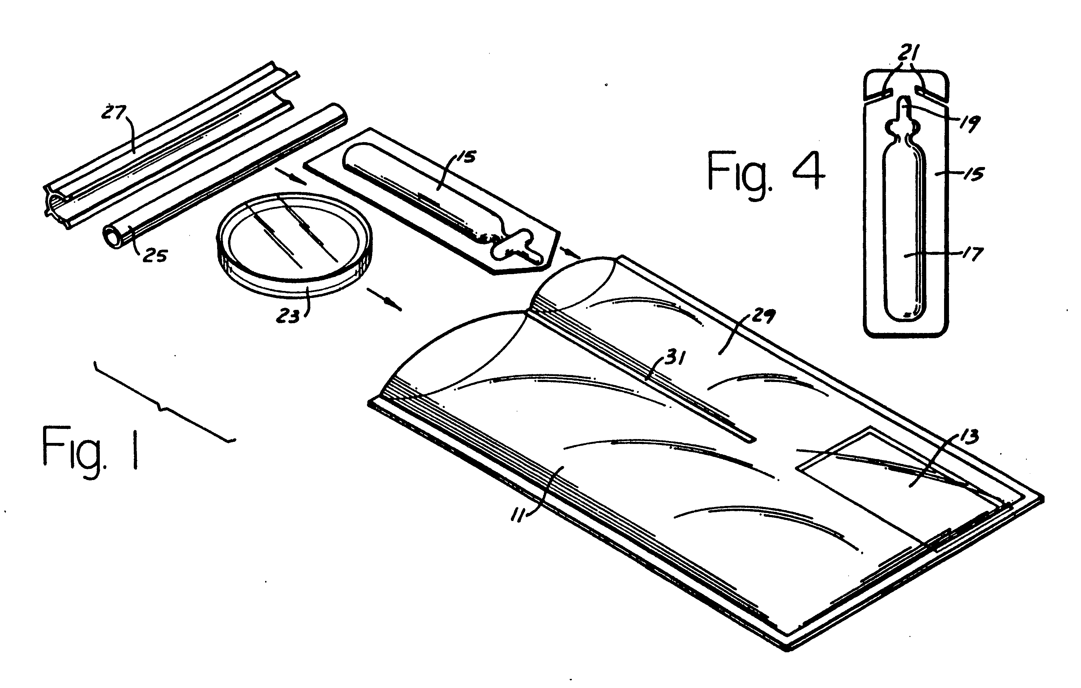

Referring now to Fig. 1, there is shown a package for removing oxygen from a gaseous atmosphere. The package is in the form of an envelope 11. Envelope 11 is made of a suitable material which is impervious to the atmosphere and moisture. Thus, for example, the envelope 11 may be formed of a laminated plastic material, such as polyvinyldichloride/polyester/polyethylene. The envelope 11 may be formed from two panels suitably secured together around the edges by heat sealing.

The interior of the envelope contains a sachet 13. In the simplest embodiment of the present invention the sachet contains only a water insoluble carbonate. In a preferred embodiment of the invention the sachet contains an admixture of a water insoluble carbonate and an oxygen reactive material. In a most preferred embodiment of the invention, the sachet contains an admixture of an oxygen reactive material, a powdered catalyst and a carbonate which can be either a water soluble carbonate or a water insoluble carbonate. The sachet 13 is secured in place within the envelope 11 by any suitable means. One suitable means is to place one edge of the sachet between the two panels of heat sealing and to heat seal the plastic over a portion of the sachet. Another suitable means would be to heat seal an area of the envelope adjacent to the sachet so as to provide an open pocket for restraining the sachet in a suitable position. The sachet is formed of a porous material, such as water resistant paper, to permit the water to penetrate to the inside of the sachet and to permit reaction of the gaseous atmosphere within the envelope with the contents of the sachet. The sachet may be overwrapped with a gas impermeable material or may be placed in a gas impermeable container for distribution to a consumer for use in anaerobic containers provided by the consumer. The sachet may also be incorporated as part of a device to transport organisms. It should be understood that the compositions of the invention can also be provided as a powdered admixture in a suitable sealed container for removal and use at an appropriate time. The compositions can also be pelleted and the pellets provided for use at an appropriate time.

For an embodiment of the invention wherein it is desired to provide an atmosphere having a predetermined level of carbon dioxide, a carbon dioxide generating composition is provided. The improved oxygen removal compositions of the present invention containing a catalyst can be used with the carbon dioxide generating compositions described in the Aswell et al patent which include a water soluble carbonate. Such carbon dioxide generating compositions include a water soluble solid acid and a water soluble carbonate in amounts suitable for generating carbon dioxide. As representative examples of suitable acids there may be mentioned: citric, tartaric, ascorbic, succinic, malic, fumaric, ethyldiamine tetracetic (EDTA) and lactic acid and the like. As representative examples of suitable carbonates, there may be mentioned: sodium bicarbonate, sodium carbonate, potassium carbonate, sodium sesquicarbonate, etc. A preferred composition includes citric acid and sodium bicarbonate. The carbon dioxide generating composition is preferably employed in the form of a powder which is 100 percent less than 200 mesh U.S. Standard Sieve Size and which is dispersed with the powdered iron.

In accordance with the present invention, however, improved results can be obtained by using a water insoluble carbonate. By the term "water insoluble carbonate" is meant those carbonate and bicarbonate salts having a solubility of less than approximately 100 milligrams per liter at 20°C. Suitable water insoluble carbonates include calcium carbonate, nickel carbonate, cobalt carbonate and magnesium carbonate (Table I).

TABLE 1

Gas Levels Using Water Insoluble Carbonates

| Carbonate | Amt(gm) | $\%CO_2$ | $\%O_2$ | $\%H_2$ |
|---|---|---|---|---|
| None | – | 0.8 | 0.4 | 24 |
| $CACO_3$ | 0.09 | 5.5 | 0.1 | 23 |
| $NiCO_3$ | 0.107 | 4.2 | 0.4 | 22 |
| $CoCO_3$ | 0.107 | 2.9 | 0.2 | 22 |

Gas levels were generated using sachets which contained the carbonates shown above at 2.4 millimoles of carbonate per liter volume of container and where the reactive metal was 0.9g iron powder (42.7 millimoles per liter), the acid was 1.15g citric acid (16 millimoles per liter), the filler was 2.86g diatomaceous earth and the activator was 3.5 ml of water. The sachet was activated in a flexible pouch plastic container with an internal volume of about 375cc when two 100 mm petri dishes are placed inside.

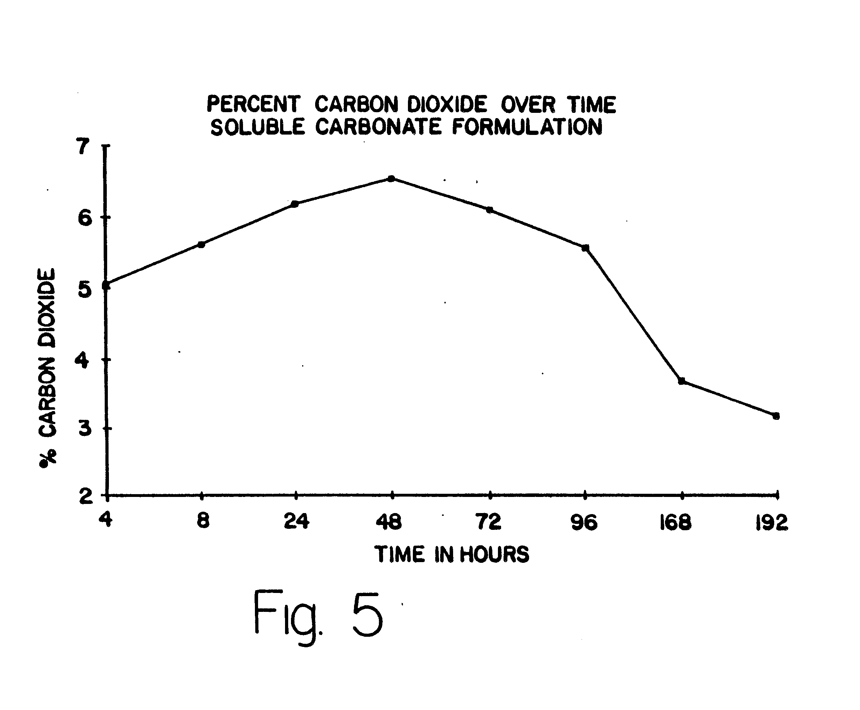

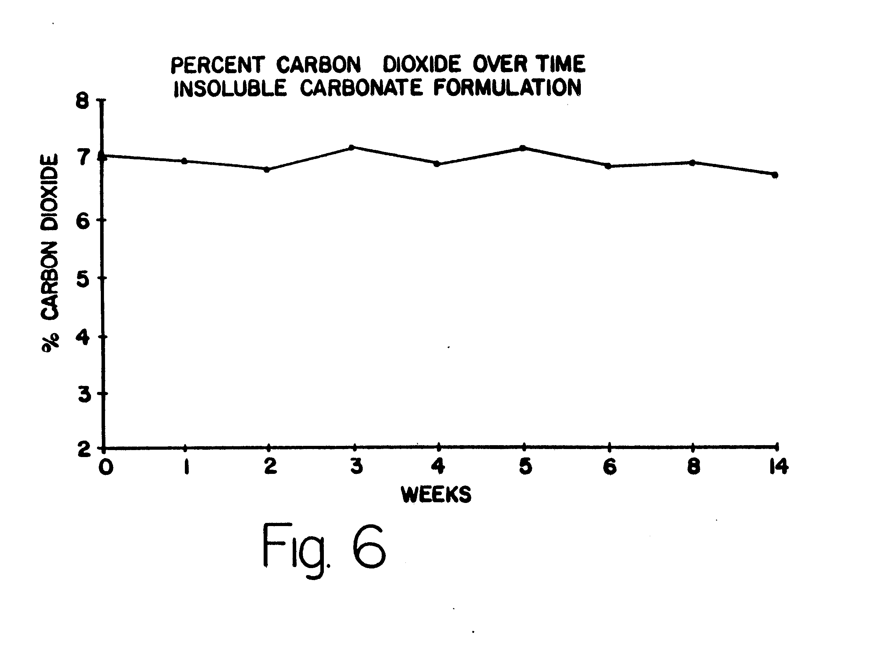

Figure 5 shows that unprotected soluble carbonate formulations rapidly lose their capacity to generate carbon dioxide gas upon exposure to the atmosphere. Figure 6, shows that insoluble carbonate formulations resist premature activation and will predictably react upon demand even when stored unprotected from ambient humidity for 14 weeks.

The use of a water insoluble carbonate is novel to generate capneic atmospheres. The Aswell et al patent refers specifically to use of a water soluble carbonate. In a preferred embodiment of the present invention a water insoluble carbonate is used. Water insoluble carbonates do not require stringent protection from the atmosphere during manufacturing or prior to use.

As indicated, the compositions may contain a material which is reactive with oxygen so as to remove oxygen from the gaseous atmosphere within the envelope. A suitable oxygen reactive material is a metal powder, wire or foil provided in a reduced state of oxygenation. Suitable metals include iron, copper, aluminum, nickel and zinc. A preferred metal is powdered iron. It has been determined that the use of iron powder having a particle size 95 percent less than 325 mesh U.S. Standard Sieve Size is a most preferred oxygen reactive material for use in the invention.

A by-product of the reaction of certain metal powders with oxygen in the presence of acidified water is the generation of hydrogen. In accordance with the present invention one use of the hydrogen generated is to help remove the oxygen faster. This is done by adding small amounts of powdered catalyst to the oxygen generating material. The added catalyst is not removed to a site in the container displaced from where the hydrogen is being generated. Also, large amounts of catalyst per liter of container volume are not used. The powdered catalyst is a noble metal catalyst, preferably powdered platinum or palladium having a surface area of from about 1 to about 50 meter$^2$/gram. The catalyst may be added to the compositions in the form of a substantially pure powdered material, however, it is preferred to use the catalyst in the form of a powdered support material. The powdered support material is preferably an inert powder coated with from about 0.5 to about 5% platinum or palladium. The inert powder is preferably less than 200 mesh U.S. Standard Sieve size. Suitable powdered inert materials include alumina, diatomaceous earth, carbonate and charcoal. These forms of powdered catalyst are all commercially available. The powdered catalyst is incorporated into the composition at a level of from about 0.1 to about 100 milligrams of powdered catalyst per liter volume of the container in which the sachet is to be used.

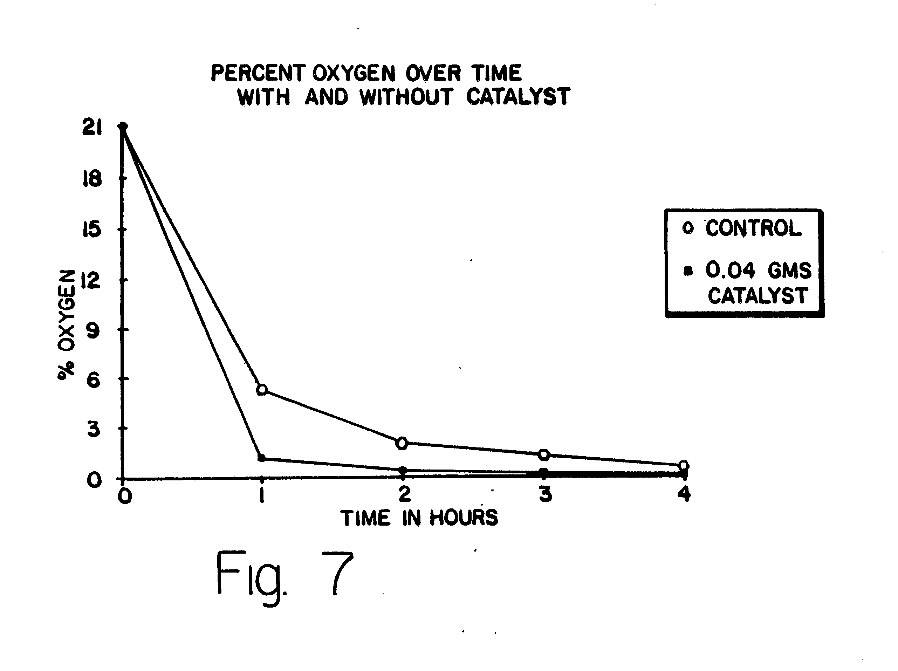

The powdered iron and the powdered catalyst act in concert to remove oxygen faster than the powdered iron can alone. As shown in Fig. 7 the Control gas oxygen level is from a sachet containing 5g iron powder, 1.4g citric acid, 6.25g diatomaceous earth and no carbonate, activated by 10 ml water in a 1.4 liter plastic disposable container. The Catalyst gas level is from the same formulation except that 0.04g of powdered palladium was added to the dry reagent mixture. The speed at which oxygen is removed is important in preserving oxygen sensitive organisms.

This is an unexpected result. The prior art teaches that the catalyst could never be added to a reagent mixture which is wetted because the catalyst is relatively inactive when wetted at room temperature. It was also discovered that no special provisions to dissipate heat or prevent ignitions (as has been attributed to finely divided catalyst) are required.

The use of the compositions of the invention has many advantages over the prior art, particularly for disposable systems. First, the rate of oxygen removal is faster than the powdered metal formulations similar to those described in the Aswell et al patent. Secondly, only a fraction of the amount of the rare earth catalyst used in the prior art borohydride systems is required to produce a noticeable effect. Thirdly, excessive heat is not generated and the reoccurring problem of melted plastic jars and bags is avoided. Finally, no separate safety shielded container with provisions to dissipate heat is required.

The carbonate, whether water soluble or water insoluble, may be combined with the previously described soluble acids or an acidified water solution may be used to activate the carbonate and the oxygen reactive material.

It has also been determined that to provide a suitable low level of oxygen required for anaerobic conditions within an appropriate time, the presence of a water absorbing inert filler is desirable. The inert filler is also desirable to provide a carrier and a dispersing agent for the components of the compositions, particularly for those compositions which use pure powdered catalyst and for those compositions which only generate a carbon dioxide atmosphere. Suitable inert fillers include diatomaceous earth, charcoal, cellulose fiber, silica gel and barium sulfate. In particular, diatomaceous earth has been found to be suitable to provide water absorptive properties and iron powder spacing properties. The filler is preferably 100 percent less than 200 mesh U.S. Standard Sieve Size. A surfactant is not required. The use of surfactants with the oxygen reactive compositions of the invention do not provide any improvement. Table II shows the gas levels that are produced in the presence of several different surfactants when they are added to an iron formulation that typically reaches about 2% oxygen at two hours. The surfactants did not appreciably increase the rate of oxygen removal when used in the oxygen reactive compositions of the invention.

TABLE II

Effect of Surfactants on Gas Levels at Two Hours

| Surfactant | Conc. | $\%O_2$ | $\%CO_2$ | $\%H_2$ |
|---|---|---|---|---|
| None | – | 2.3 | 7.9 | 16 |
| Triethanolamine Sulfonate | 1% | 2.7 | 7.3 | 16 |
| (Ultrawet) | 0.1% | 2.1 | 7.4 | 17 |
| Triton X-100 | 1% | 2.6 | 7.9 | 17 |
| | 0.1% | 2.1 | 7.9 | 19 |
| Zwittergent | 1% | 2.6 | 7.7 | 17 |
| | 0.1% | 1.9 | 8.1 | 20 |
| Sodium Dodecyl Sulfate | .33g | 1.5 | 7.9 | 18 |
| | .01g | 2.5 | 8.0 | 17 |

Gas levels were produced by two sachets having a total of 10g iron powder (72 millimoles per liter), 9g citric acid (16.7 millimoles per liter), 1g calcium carbonate (4 millimoles per liter) and 10g diatomaceous earth (4g per liter) activated by 10 ml water containing the surfactants above. The sachets were preliminarily packaged in a gas impermeable package. They were removed from the package as required and placed in the bottom of a fixed wall reusable container with an internal volume of 2.5 liters.

Those compositions of the invention which are useful for providing capneic atmospheres are admixtures of an insoluble carbonate, a solid acid and an inert filler. The insoluble carbonate is present at a level of from about $1\times10^{-3}$ to about $1\times10^{-2}$ moles, preferably from about $1\times10^{-3}$ to about $6\times10^{-3}$ moles, per liter of volume of the container in which the composition will be used. The term "mole", as used herein, is used in the conventional manner to denote a gram-mole. The soluble acid is present at a level of from about .1 to about 100 moles, preferably from about .6 to about 60 moles, per mole of carbonate. The inert filler is present at a level of about 1 to about 50, preferably from about 5 to about 30, grams per gram of carbonate used.

Those compositions of the invention which are useful for reducing oxygen but which do not contain a powdered catalyst are admixtures of a powdered metal, an insoluble carbonate, a soluble acid and an inert filler. The powdered metal is present at a level of from about $9\times10^{-2}$ to about $4\times10^{-1}$, preferably from about $4\times10^{-3}$ to about $9\times10^{-2}$ moles, moles per liter of container volume, the insoluble carbonate is present at a level of from about $1\times10^{-3}$ to about $1\times10^{-2}$; preferably from about $1\times10^{-3}$ to about $6\times10^{-3}$ moles of carbonate per liter of container volume, the soluble acid is present at a level of from about .1 to about 100, preferably from about .5 to about 40, moles per mole of insoluble carbonate and the inert filler is present at a level of from about 10 to 100, preferably from about 20 to about 80, grams per gram of insoluble carbonate.

Those compositions of the invention which are useful for reducing oxygen and which contain a powdered catalyst are an admixture of a powdered metal, a powdered catalyst, a solid acid and an inert filler. These compositions do not require a carbonate. The use of a carbonate is optional and when a carbonate is used it may be either an insoluble carbonate or a soluble carbonate. The powdered metal is present at a level of from about $4\times10^{-3}$ to about $4\times10^{-1}$, preferably from about $4\times10^{-3}$ to about $9\times10^{-2}$, moles per liter of container volume, the powdered catalyst is present at a level of from about 0.1 to about 5, preferably from about 0.5 to about 3, milligrams of catalyst metal per liter of container volume, the soluble acid is present at a level of from about 0.05 to about 4, preferably from about 0.1 to about 3, moles per mole of powdered metal and the inert filler material is present at a level of from about 0.5 to about 40, preferably from about 1 to about 25, grams per gram of powdered metal. If a carbonate is used, whether soluble or insoluble, the carbonate is present at a level of from about $1\times10^{-3}$ to about $1\times10^{-2}$, preferably from about $1\times10^{-3}$ to about $6\times10^{-3}$, moles of carbonate per liter of volume.

The goal of the present invention is to attain the appropriate carbon dioxide and oxygen gas levels in the container as rapidly as possible. To achieve this goal, it has been determined that the level of water added to the composition of the invention is important. The water should be added to the composition at a level of from about 50 to about 250 percent of the weight of the inert filler material in the composition. In one embodiment of the invention, the oxygen reactive composition in the sachet can be premoistened with a suitable level of water prior to sealing of the package without insertion of the petri dish or dishes. The oxygen within the package is removed by reaction with the oxygen reactive material, which retains a residual ability to react with additional oxygen. The package can then subsequently be reopened for insertion of the specimen and reaction with the oxygen introduced by the reopening of the package.

In a preferred embodiment of the invention, a suitable predetermined level of water is provided for use with the compositions of the invention as a separate vial, 15, as shown in Fig. 4. The water containing vial 15 is made of heat sealed plastic having a tube 17 shaped therein. A spout 19 is provided at the end of the tube. The spout can be opened by use of the tear strip 21 provided in the vial. Alternatively, the water may be added to a suitable container provided with a sachet by any suitable means, such as a pipette.

In use, as shown in Fig. 1, the end of the vial is torn from the vial to expose the tip of the spout and the vial is inserted within the envelope 11 into compartment 29 in fluid communication with the sachet containing the oxygen reactive material. One or more petri dishes 23 are inserted in the container and the container is sealed.

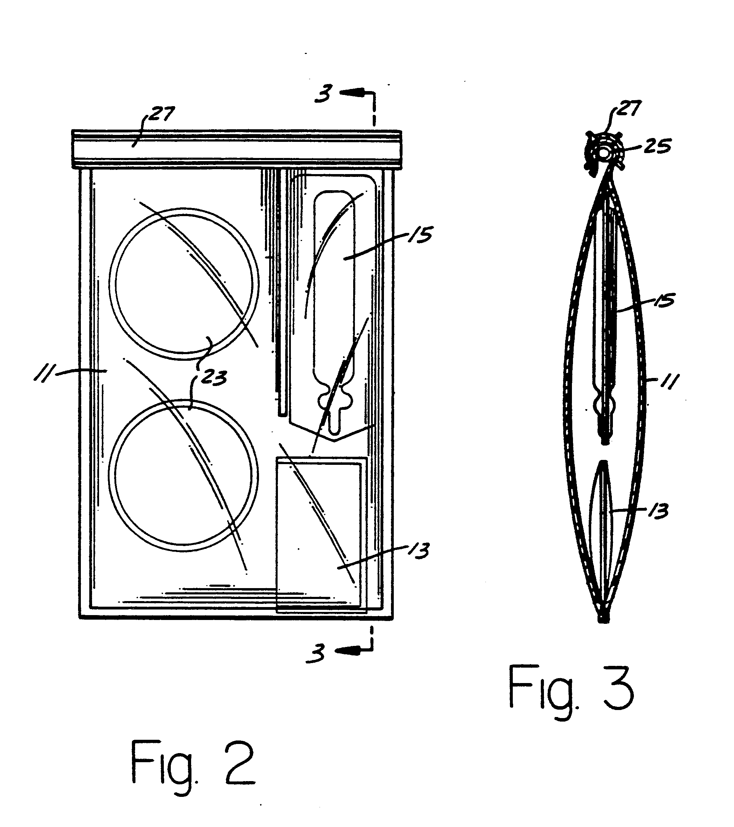

A suitable means for sealing a flexible envelope is shown in Figs. 1 and 2. As shown in Fig. 2, the open end of the envelope 11 is wrapped around tubular rod 25. A semi-circular clamping rod 27 is clamped over the end of the envelope and the rod 25, as shown in Fig. 2. The water is then dispensed from the vial by squeezing the vial, while it is in place in the envelope. Thereafter, the package is placed in a suitable incubation temperature for generation of any oxygen free atmosphere and growth of the anaerobic organism.

In a commercial embodiment of the present invention, a plurality of envelopes having the sachet suitably positioned in the envelope would be packaged with a matching plurality of vials containing a predetermined level of water.

It should be understood that the package described in the present specification is merely exemplary of one type of container in which the composition of the present invention may be used. As previously indicated, the compositions may be enclosed with a sachet, may be provided as a loose, bulk material, or may be compacted into a pellet. In whatever form the compositions are provided they are suitably protected from the ambient atmosphere prior to use.

The following examples further illustrate various features of the present invention but are intended to in no way limit the scope of the invention which is defined in the appended claims.

EXAMPLE I

A carbon dioxide producing composition for generation a capneic atmosphere per liter volume of container is prepared by blending together the following materials:

| Material | Amount |
|---|---|
| calcium carbonate | 2.7 millimoles |
| diatomaceous earth | 5.3 g |

Sachets were prepared having 2.1 grams of the above blend using porous heat-sealable paper similar to that used for tea bags.

The sachets were placed in position in a heat-sealable plastic bag having an internal volume of about 375 cc. The sachets were activated with 5 mls of a 10%(w/v) citric acid solution (6.9 millimoles per liter citric acid), two 100 mm petri dishes were placed along side the sachet in the bag and the bag was sealed shut.

A Hewlett Packard gas chromatograph model 5880A was used to determine gas concentrations. It was equipped with thermal conductivity detectors. The oven temperature was maintained at 66°C. An 8 foot by 1/8th inch O. D. Molecular Sieve 5A column (80/100 mesh) and an 8 foot by 1/8th inch O. D. Porapak Q column (80/100 mesh) were installed in series. Argon was used as the carrier gas. Flow rate through the column was 30ml/min. A column switching system was used to allow simultaneous measurements of carbon dioxide, hydrogen, oxygen, nitrogen and water. The data was analyzed using an HP5880A integrator terminal which, after calibration, supplied the retention time, peak area, and calculated percent for each of the five gases.

The test system was activated and placed at 35°C for the designated time interval. A two centimeter square piece of 3M double-sided tape was applied to each system to provide a platform for gas sampling. The tape platform minimizes the size of the sampling hole made by the syringe and prevents tearing of the incubation bag. The atmosphere was collected with a 10 ml syringe or an automatic sampling device. Samples were injected using a one ml sample loop.

The carbon dioxide level in three bags was measured to be 7.2, 7.1 and 6.7 percent by volume after 1 hour.

EXAMPLE II

An oxygen reducing, carbon dioxide generating composition for creating a microaerophilic atmosphere per liter volume of container is prepared by blending together the following materials:

| Material | Amount |
|---|---|
| iron powder (325 mesh) | 8.3 millimoles |
| calcium carbonate | 2.7 millimoles |
| citric acid | 8 millimoles |
| diatomaceous earth (Mansville Standard SuperCel) | 11g |

Sachets were prepared having 5.0 grams of the above blend using porous heat-sealable paper similar to that used for tea bags.

The sachet were placed in position in a heat-sealable plastic bag having an internal volume of about 375 cc. The sachets were activated with 5 mls of water, two 100 mm petri dishes were placed along side the sachet in the bag and the bag was sealed shut. The gas levels were determined using the procedure in Example I. For a set of fifteen samples the carbon dioxide level in the bags after 1 hour was found to average 7.9 percent by volume and had a standard deviation of 0.6 percent. The oxygen concentration was found to average 9.9 percent and had a standard deviation of 1.0 percent.

EXAMPLE II

An oxygen reducing, carbon dioxide generating composition for creating an anaerobic atmosphere per liter volume of container is prepared by blending together the following materials:

| Material | Amount |
|---|---|
| iron powder (325 mesh) | 64 millimoles |
| calcium carbonate | 3.2 millimoles |
| citric acid | 12 millimoles |
| diatomaceous earth (Mansville Standard SuperCel) | 4.5g |

Sachets were prepared having 15.0 grams of the above blend using porous heat-sealable paper similar to that used for tea bags.

Six 100 mm petri dishes were placed in a heat-sealable plastic bag having an internal volume of about 1.4 liters, a sachet was placed in a plastic tray on top of the dishes, the sachet was activated with 10 mls of water and the bag was sealed shut. The gas levels were determined using the procedure in Example I. For a set of fifteen samples, the carbon dioxide level in the bags after 2 hours was found to average 6.6 percent by volume and had a standard deviation of 0.3 percent. The oxygen averaged 0.2 percent by volume and had a standard deviation of 0.1 percent.

EXAMPLE IV

A more rapid oxygen reducing, carbon dioxide generating composition for creating an anaerobic atmosphere per liter volume of container is prepared by blending together the following materials:

| Material | Amount |
|---|---|
| iron powder (325 mesh) | 64 millimoles |
| calcium carbonate | 3.2 millimoles |
| citric acid | 12 millimoles |
| diatomaceous earth | 4.5g |
| (Mansville Standard SuperCel) | |
| 5% palladium coated powdered alumina | 28.5mg |

Sachets were prepared having 15.0 grams of the above blend using porous heat-sealable paper similar to that used for tea bags.

Six 100 mm petri dishes were placed in a heat-sealable plastic bag having an internal volume of about 1.4 liters, a sachet was placed in a plastic tray on top of the dishes, the sachet was activated with 10 mls of water and the bag was sealed shut. The carbon dioxide level in the bag after 2 hours was found to average 7.2 percent by volume and had a standard deviation of 0.2 percent. The oxygen averaged 1.5 percent at 1 hour and 0.2 percent at 2 hours and had a standard deviation of 0.03 percent.

Major improvements over prior art formulations are provided by the compositions of this invention: stability and reliability through the use of water insoluble carbonates, small quantities of metal powder to reduce but not eliminate oxygen for certain types of microorganisms; small quantities of powdered material coated with catalyst in the wetted sachet to produce an unanticipated, significant acceleration of oxygen removal and to allow a lower level of metal powder to be functional.

**Claims**

1. A composition for producing an oxygen reduced environment suitable for the growth of anaerobic microorganisms in an enclosed container having a predetermined volume, said composition comprising:
    (a) an oxygen reactive powdered metal and
    (b) a powdered noble metal catalyst.

2. A composition in accordance with Claim 1 which also includes a soluble acid.

3. A composition in accordance with Claim 1 which also includes a carbonate, and said carbonate is selected from the group consisting of water soluble carbonates and water insoluble carbonates.

4. A composition in accordance with Claim 1 wherein said powdered metal is selected from the group consisting of iron, aluminum, copper, nickel and zinc.

5. A composition in accordance with Claim 2 wherein said powdered metal is present at a level of from about $4 \times 10^{-3}$ to about $4 \times 10^{-1}$ moles per liter of container volume, said powdered catalyst is present at a level of from about 0.1 to about 5 milligrams per liter volume of container and said soluble acid is present at a level of from about 0.05 to about 4 moles per mole of powdered metal.

6. A composition in accordance with Claim 3 wherein said carbonate is present at a level of from about $1 \times 10^{-3}$ to about $1 \times 10^{-2}$ moles of carbonate per liter of container volume.

7. A composition in accordance with Claim 1 wherein said powdered catalyst is selected from the group consisting of platinum and palladium.

8. A composition in accordance with Claim 1 wherein said powdered catalyst is coated onto an inert particle.

9. A composition in accordance with Claim 9 wherein said inert particle is selected from the group consisting of alumina, diatomaceous earth, carbonate and charcoal and said inert powder has a particle size of less than about 200 mesh U. S. Standard Sieve size.

10. A composition in accordance with Claim 8 wherein said powdered catalyst is present on the surface of said inert particle at a level from about 0.5 to about 5% by weight.

11. A composition in accordance with Claim 1 wherein powdered catalyst has a surface area of from about 1 to about 50 meter$^2$/gram.

12. A composition in accordance with Claim 1 which is dispersed within a gas permeable sachet.

13. A composition for producing an oxygen reduced, carbon dioxide enriched environment suitable for the growth of microorganisms in an enclosed container having a predetermined volume, said composition comprising:

(a) a water insoluble carbonate;

(b) a water soluble acid; and

(c) an oxygen reactive powdered metal.

14. A composition in accordance with Claim 13 wherein said powdered metal is present at a level of from about $4x10^{-3}$ to about $44x10^{-1}$ moles of powdered metal per liter of container volume, said insoluble carbonate is present at a level of from about $1x10^{-3}$ to about $1x10^{-2}$ moles of carbonate per liter of container volume and said soluble acid is present at a level of from about .1 to about 100 moles per moler of insoluble carbonate.

15. A composition in accordance with Claim 13 which is disposed within a gas and liquid permeable sachet.

16. A composition in accordance with Claims 10 or 13 which is provided in the form of a pellet.

17. A composition for producing a carbon dioxide enriched environment suitable for the growth of microorganisms in an enclosed container having a predetermined volume, said composition comprising:

(a) a water insoluble carbonate;

(b) a water soluble acid; and

(c) an inert filler material.

18. A composition in accordance with Claim 13 or 17 wherein said water insoluble carbonate is selected from the group consisting of calcium carbonate, magnesium carbonate, zinc carbonate and cobalt carbonate.

19. A composition in accordance with Claims 13 or 17 wherein said water soluble acid is selected from the group consisting of citric, tartaric, ascorbic, succinic, malic, fumaric, EDTA and lactic acid.

20. A composition in accordance with Claim 17 wherein said water insoluble carbonate is present at a level of from about $1x10^{-3}$ to about $1x10^{-2}$ moles of carbonate per liter of volume of said container, said water soluble acid is present at a level of from about 0.1 to about 100 moles of acid per mole of insoluble carbonate and said inert filler material is present at a level of from about 1 to about 50 grams per gram of insoluble carbonate used.

11

Fig. 4

Fig. 1

Fig. 2

Fig. 3

# PERCENT CARBON DIOXIDE OVER TIME
# SOLUBLE CARBONATE FORMULATION

Fig. 5

PERCENT CARBON DIOXIDE OVER TIME
INSOLUBLE CARBONATE FORMULATION

% CARBON DIOXIDE (y-axis, values 2 through 8)

WEEKS (x-axis, values 0, 1, 2, 3, 4, 5, 6, 8, 14)

Fig. 6

PERCENT OXYGEN OVER TIME
WITH AND WITHOUT CATALYST

Legend:
- o CONTROL
- ■ 0.04 GMS CATALYST

Y-axis: % OXYGEN
X-axis: TIME IN HOURS

Fig. 7

0 290 781